# EUROPÄISCHE PATENTSCHRIFT

(11) **EP 0 359 900 B1**
(45) Veröffentlichungstag und Bekanntmachung des Hinweises auf die Patenterteilung: **19.05.1993**
(21) Anmeldenummer: 89105590.7
(22) Anmeldetag: 30.03.1989
(51) Int. Cl.: C12P 1/00, C12M 1/12

(54) **Verfahren zum Abtrennen von hochmolekularen Substanzen aus flüssigen Nährmedien sowie Vorrichtung zur Durchführung des Verfahrens**
Process for separating highly molecular substances from liquid culture media, and device for carrying out the process
Procédé de séparation de substances de haut poids moléculaire à partir de milieux nutritifs liquides ainsi qu'une installation pour la mise en oeuvre du procédé

(30) Priorität: 19.09.1988 DE 3831771
(43) Veröffentlichungstag der Anmeldung: 28.03.1990
(73) Patentinhaber: Heraeus Sepatech GmbH, D-37502 Osterode am Harz (DE)
(72) Erfinder: Schmeisser, Holger, Dr., D-3400 Göttingen (DE); Köhn, Heinz-Gerhard, Dr., D-3402 Dransfeld (DE)
(74) Vertreter: Heinen, Gerhard, Dr.

(56) Entgegenhaltungen:
- DE-A- 3 840 447
- CHEMICAL ABSTRACTS, Band 109, 1988, Seite 114, Zusammenfassung Nr. 95251p, Columbus, Ohio, US; & JP-A-63 107 710 (ASAHI CHEMICAL INDUSTRY CO., LTD) 12-05-1988

## Beschreibung

Die Erfindung betrifft ein Verfahren zum Abtrennen von Feststoffen, z.B. Bakterien, Hefen, tierischen und/oder menschlichen Zellen, aus flüssigen Nährmedien und Anreicherung von in der verbleibenden Flüssigkeit enthaltenen hochmolekularen Substanzen, wobei das flüssige Nährmedium aus einem Fermenter oder Vorratsgefäß entnommen und durch Zentrifugation in seine Feststoffe und flüssigen Bestandteile getrennt wird und in einem weiteren Verfahrensschritt durch Filtrierung in einer Filtereinheit die hochmolekularen Substanzen in den flüssigen Bestandteilen angereichert werden. Weiterhin betrifft die Erfindung eine Vorrichtung zur Durchführung des Verfahrens mit einem das flüssige Nährmedium enthaltenden Fermenter oder Vorratsgefäß, der über eine erste Pumpe strömungsmäßig mit dem Durchlaufrotor einer Zentrifuge verbunden ist, die einen Ablauf aufweist, der in einen Behälter führt.

Solche Verfahren bzw. entsprechende Vorrichtungen sind allgemein bekannt. Sie werden zur sogenannten Zellernte eingesetzt, d.h. zum Abtrennen und Aufkonzentrieren von beispielsweise Bakterien aus flüssigen Nährmedien. Hierzu werden Zentrifugen, insbesondere Ultra-Zentrifugen, verwendet, die im Durchfluß betrieben werden. In dem Zentrifugenrotor werden Feststoffe gesammelt und die flüssigen Bestandteile aufgefangen. Auf diese Art und Weise werden sehr große Mengen an Nährmedien zentrifugiert und in einem Vorratsgefäß gesammelt. Die von den Feststoffen abgetrennten flüssigen Bestandteile werden anschließend in einem gesonderten Verfahren bzw. einer gesonderten Vorrichtung über sogenannte Membran-Trenn-Verfahren in die hochmolekularen und niedermolekularen Bestandteile getrennt. Für die Filtrationstechnik werden verschiedene Filterarten eingesetzt, beispielsweise Ultra-Filter oder Mikro-Filter, die sich durch die verwendeten Membrane unterscheiden, wobei durch mikroporöse Membrane Bestandteile im 1/10 µm Bereich, z.B. Bakterien, abgetrennt werden, während mit den Ultra-Membranen wesentlich kleinere Bestandteile, beispielsweise Eiweiße, abtrennbar sind.

Die bekannten Verfahren sind dahingehend als nachteilig anzusehen, daß sie mehrere voneinander getrennte Verfahrensschritte beinhalten, deren Verfahrensparameter jeweils für sich eingestellt und so nicht oder nur ungenügend aufeinander abgestimmt werden können. Außerdem sind diese Verfahren bzw. die entsprechenden Vorrichtungen mit dem Nachteil verbunden, daß die Behälter mit den Bestandteilen, die zu zentrifugieren bzw. zu filtern sind, umgesetzt werden müssen.

Der vorliegenden Erfindung liegt nun die Aufgabe zugrunde, ein Verfahren und eine Vorrichtung zum Abtrennen von hochmolekularen Substanzen aus flüssigen Nährmedien anzugeben, das die vorstehenden Nachteile vermeidet und einen kontinuierlichen Ablauf der Zentrifugation und Filtration ermöglicht.

Gelöst wird diese Aufgabe hinsichtlich des eingangs beschriebenen Verfahrens dadurch, daß zentral geregelt der Durchlaufrotor einer Zentrifuge kontinuierlich aus dem Fermenter oder Vorratsgefäß mit flüssigem Nährmedium beschickt wird, bis eine vorgegebene als Sollwert zentral gespeicherte Menge der flüssigen Bestandteile von der Zentrifuge in ein Zwischenreservoir abgegeben ist, und daß diese flüssigen Bestandteile kontinuierlich direkt der Filtereinheit zugeführt werden mit einer anschließenden Rückführung der hochmolekularen Bestandteile in das Zwischenreservoir, wobei der Filtrationsdruck zentral überwacht und in Abhängigkeit der Pumpleistung einer der Filtereinheit zugeordneten Pumpe eingestellt wird und wobei die Beschickung der Zentrifuge innerhalb vorgegebener Werte des momentanen Füllstandes des Zwischenreservoirs geregelt wird. Bei einer Vorrichtung wird die Aufgabe dadurch gelöst, daß der Behälter als Zwischenreservoir dient und über eine Zuleitung und eine Rückführungsleitung mit einer Ultra-Filtereinheit verbunden ist, wobei in der Zuleitung zu der Filtereinheit eine zweite Pumpe angeordnet ist, und daß die erste Pumpe, die zweite Pumpe sowie der Antrieb der Zentrifuge über Steuerleitungen mit einer zentralen Rechen- und Regeleinheit verbunden sind. Dem Zwischenreservoir, das über den Durchlaufrotor der Zentrifuge kontinuierlich nachgefüllt wird, wird unmittelbar Flüssigkeit entnommen und der Filtereinheit zugeführt. Gleichzeitig dient aber das Zwischenreservoir, das über eine Rückführung mit der Filtereinheit verbunden ist, als Behälter, in dem hochmolekulare Substanzen, an denen Interesse besteht, in den flüssigen Bestandteilen angereichert werden. Da mit zunehmender Anreicherung der hochmolekularen Bestandteile die Abnahme an flüssigen Bestandteilen im Zwischenreservoir geringer wird, wird der Durchlaufrotor derart gesteuert, daß mit sich einpendelndem Füllstand im Zwischenreservoir die Nachführung an flüssigen Bestandteilen aus dem Durchlaufrotor der Zentrifuge geringer wird. In dieser Anordnung kann, in Abhängigkeit des Füllvolumens des Zwischenreservoirs, die Zentrifugation und Filtration derart gesteuert und geregelt werden, daß in dem Zwischenreservoir eine gewünschte Menge an Flüssigkeit angereichert mit hochmolekularen Substanzen angesammelt werden kann, wobei die Höhe der Konzentration an hochmolekularen Substanzen zu jedem Zeitpunkt des Verfahrens festgelegt werden kann. Verluste oder verbleidende Mengen an Flüssigkeiten treten nicht ein. Da alle Komponenten, d.h. der Zentrifugenrotor, die Pumpen sowie die Filtereinheit und die dazugehörigen Ventile und Drucksensoren, zentral überwacht und angesteuert werden, kann das Verfahren in allen Verfahrensparametern abgestimmt werden. So wird im bevorzugten Ausführungsformen des Verfahrens der momentane Füllstand in dem Zwischenreservoir ständig zentral als Ist-Größe überwacht und mit der eingestellten Soll-Größe verglichen, um die Zuflußrate von dem Durchlaufrotor der Zentrifuge in das Zwischenreservoir anzupassen. In einer sehr einfachen Ausführungsform kann eine solche Füllstandsüberwachung über eine elektronische Waage erfolgen, die beispielsweise mit einer zentralen Rechen- und Steuereinheit in Verbindung steht. Zu Beginn eines jeden Zentrifugationsvorganges sollte die Waage tariert oder aber der Wert des leeren, als Zwischenreservoir dienenden Behälters als Null- oder Ausgangswert zentral gespeichert werden.

Da Durchlaufrotoren, die zur Trennung der Feststoffe von den flüssigen Bestandteilen eingesetzt werden, nur ein begrenztes Aufnahmevolumen für die festen Bestandteile, die in dem Rotor verbleiben, aufweisen, ist es von Vorteil, den Feststoffgehalt der in das Zwischenreservoir überführten flüssigen Bestandteile ständig zu ermitteln und bei Überschreiten eines vorgegebenen Grenzwertes den Zufluß an flüssigem Nährmedium zu dem Durchlaufrotor zu unterbrechen, da dann davon auszugehen ist, daß der Durchlaufrotor mit Feststoffen belegt ist, so daß er quasi überläuft. Um eine optimale Einstellung der Filtration an der Filtereinheit zu gewährleisten, sollte die Pumpleistung der der Filtereinheit zugeordneten Pumpe derart geregelt werden, daß ein zentral als Sollwert gespeicherter Filtrationsdruck, verglichen mit dem Druck gebildet aus dem arithmetischen Mittel zwischen dem Druck eingangsseitig der Filtereinheit und dem Druck an dem die hochmolekularen Substanzen abführenden Ausgang der Filtereinheit nicht überschritten wird. Mit einer solchen Verfahrensweise wird die Filtereinheit mit ihrem maximalen Wirkungsgrad betrieben und gleichzeitig überwacht, daß die Filtereinheit nicht überbeansprucht wird.

Das Ende des Verfahrens kann dadurch angegeben werden, daß beim Überschreiten einer vorgegebenen, zentral gespeicherten, maximalen Menge an flüssigen Bestandteilen in dem Zwischenreservoir der Fluß eingangsseitig und ausgangsseitig der Filtereinheit unterbrochen wird. Gleichzeitig wird der Zufluß aus dem Durchlaufrotor in das Zwischenreservoir gestopt, da eine ausreichende Sättigung der in dem Zwischenreservoir eingefüllten flüssigen Bestandteile mit hochmolekularen Substanzen erreicht ist.

Eine weitere Möglichkeit, den Druck im Bereich der Filtereinheit zu beeinflussen, ist durch die Regelung einer zusätzlichen Drossel an dem die hochmolekularen Substanzen enthaltenden Ausgang der Filtereinheit möglich. Mit dieser Drossel kann ein Staudruck erzeugt und damit die Strömungsgeschwindigkeit durch die Filtereinheit herabgesetzt werden.

Da Filter zu Verstopfungen neigen, ist es von Vorteil, die Filtereinheit zu Reinigungszwecken in größeren Zeitabständen im Gegenstrom mit aus dem Zwichenreservoir entnommener Flüssigkeit zu durchspülen. Für eine solche Spülung weist die Vorrichtung ausgangsseitig der zweiten Pumpe, die die flüssigen Bestandteile der Filtereinheit zuführt, eine Bypass-Leitung auf, die über ein darin eingesetztes Bypass-Ventil absperrbar ist und über die der Filtereinheit im Gegenstrom flüssige Bestandteile zugeführt werden können. Da sich durch eine solche Rückführung die Druckverhältnisse in den Leitungen zu und von der Filtereinheit ändern können und normale Absperrventile eine ausreichende Dichtigkeit nun in einer Richtung gewährleisten, ist ausgangsseitig der zweiten Pumpe in der Zuleitung zu der Filtereinheit nach der Abzweigung der Bypass-Leitung ein Rückstau-Ventil angeordnet, das entgegen der Pumprichtung der zweiten Pumpe wirkt und einen Rückfluß an flüssigen Bestandteilen von der Leitung eingangsseitig der Filtereinheit unterbindet, falls ein solcher Rückstau bei der Spülung der Filtereinheit auftritt. Die Druckverhältnisse eingangsseitig und ausgangsseitig der Filtereinheit werden über zwei Drucksensoren in der Zuleitung und der Rückführungsleitung der Filtereinheit überwacht, die die momentanen Druckwerte ständig einer zentralen Rechen- und Regeleinheit zuführen. Für die Überwachung des Feststoffgehaltes der aus dem Durchlaufrotor der Zentrifuge in das Zwischenreservoir überführten flüssigen Bestandteile kann ein optischer Sensor, der ebenfalls mit der Rechen- und Regeleinheit verbunden ist, angeordnet werden.

Um das Trennverfahren gezielt beenden zu können, sind Absperrventile in der Zuleitung zu der Filtereinheit sowie in der Rückführungsleitung zwischen der Filtereinheit und dem Zwischenreservoir von Vorteil.

Das erfindungsgemäße Verfahren sowie die erfindungsgemäße Vorrichtung werden anhand der Zeichnung nachfolgend beschrieben. In der Zeichnung zeigt
- Figur 1: schematisch den Aufbau einer erfindungsgemäßen Vorrichtung,
- Figur 2: ein Flußdiagramm mit einem Verfahrensablauf, wie er mit der Vorrichtung nach Figur 1 durchgeführt werden kann,
- Figur 3: das "Unterprogramm 1" für den Verfahrensablauf nach Figur 2 und
- Figur 4: das "Unterprogramm 2" für den Verfahrensablauf nach Figur 2.

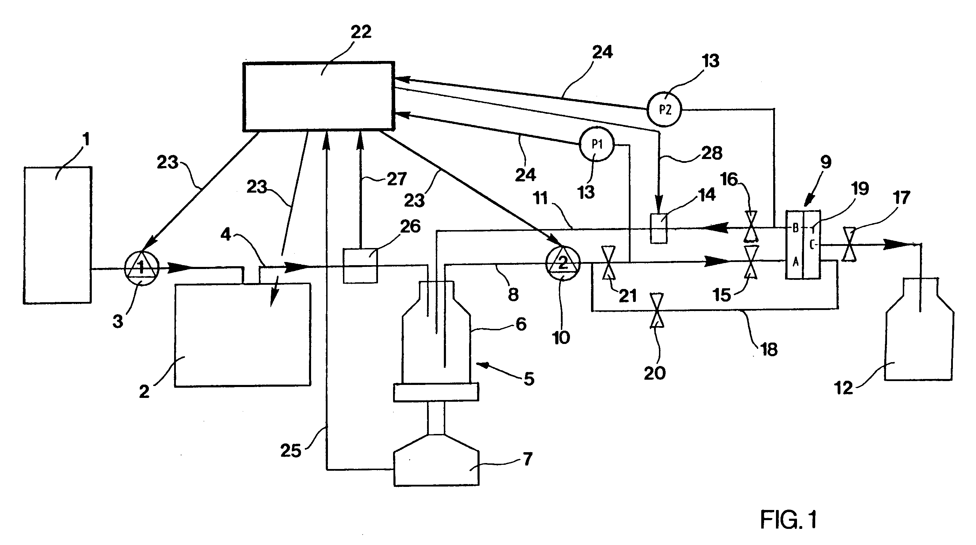

Wie der Figur 1 zu entnehmen ist, weist die Vorrichtung einen Fermenter 1 auf, in dem Nährflüssigkeit bevorratet ist. Dieser Fermenter 1 ist mit dem Durchlaufrotor einer Zentrifuge 2 über eine erste Pumpe 3 verbunden, so daß dem Durchlaufrotor der Zentrifuge 2 kontinuierlich Nährflüssigkeit zugeführt werden kann. Der Ablauf 4 der Zentrifuge 2 führt in ein Zwischenreservoir 5 in Form eines Behälters 6, der auf einer elektronischen Waage 7 steht. Mit der elektronischen Waage 7 wird ständig die Gewichtszu- oder -abnahme in dem Zwischenreservoir 5 beoachtet. Das Zwischenreservoir 5 ist über eine Zuleitung 8 mit einer Filtereinheit 9 verbunden. Über diese Zuleitung 8 wird mittels einer zweiten Pumpe 10 in der Zuleitung der Filtereinheit 9 Flüssigkeit zugeführt, wobei in der Filtereinheit 9 die hochmolekularen Substanzen getrennt und über die Rückführungsleitung 11 dem Zwischenreservoir 6 wieder zugeführt werden. Die von hochmolekularen Substanzen gereinigte Flüssigkeit wird von der Filtereinheit 9 an einen Behälter 12 abgegeben. In der Filtereinheit 9 ist der Eingang, der mit der Zuleitung 8 verbunden ist, mit A, der Ausgang, der mit der Rückführungsleitung 11 verbunden ist, mit B, und der Ausgang, der die Flüssigkeit an den Behälter 12 abgibt, mit C bezeichnet. Sowohl in der Zuleitung 8 als auch in der Rückführungsleitung 11 ist jeweils ein Drucksensor 13, 14 angeordnet, um über diese Leitungen 8, 11 den Druck in der Filtereinheit 9 zu überwachen und gegebenenfalls einstellen zu können. Für eine solche Druckeinstellung ist in der Rückführungsleitung 11 eine Drossel 14 eingesetzt. Sowohl in der Zuleitung 8 als auch in der Rückführungsleitung 11 ist unmittelbar vor bzw. nach dem Eingang A bzw. Ausgang B der Filtereinheit 9 jeweils ein Absperrventil 15, 16 eingesetzt. Ebenso ist dem Ausgang C für die von hochmolekularen Substanzen gereinigte Flüssigkeit der Filtereinheit ein weiteres Absperrventil 17 zugeordnet, mit dem der Fluß zu dem Behälter 12 unterbrochen werden kann.

Um den Filter im Gegenstrom reinigen zu können, dient eine Bypass-Leitung 18, die von der Zuleitung 8 auf der Druckseite der zweiten Pumpe 10 zur Filtereinheit 9 führt und Flüssigkeit aus der Zuleitung der Filtereinheit 9 entsprechend der unterbrochenen Linie 19 zuführt, diesen also im Gegenstrom durchspült, die dann über den Ausgang B und der Rückführungsleitung 11 in das Zwischenreservoir 5 zurückgeführt wird. Auch diese Bypass-Leitung 18 ist über ein Bypass-Ventil 20 absperrbar, das nur dann geöffnet wird, wenn die Filtereinheit 9 im Gegenstrom durchspült werden soll. Von der zweiten Pumpe 10 aus gesehen nach der Stelle, an der die Bypass-Leitung abzweigt, ist in die Zuleitung 8 ein Rückstau-Ventil 21 eingesetzt, das entgegen der normalen Strömugnsrichtung der Flüssigkeit in der Zuleitung 8, die durch die Pfeile angedeutet ist, wirkt und verhindern soll, daß beim Spülen der Filtereinheit 9 sich ein Gegendruck in der Zuleitung 8 aufbaut. Ein solches Rückstau-Ventil 21 ist notwendig, da üblicherweise solche Absperrventile eine sichere Dichtung in einer Strömungsrichtung gewährleisten. Die einzelnen Komponenten der Vorrichtung werden über eine zentrale Rechen- und Regeleinheit 22 überwacht und der Verfahrensablauf geregelt, wobei die erste Pumpe 3, die zweite Pumpe 10 und der Antrieb der Zentrifuge 2 über Steuerleitungen 23 mit der Rechen-und Regeleinheit 22 in Verbindung stehen; auch die beiden Drucksensoren 13 sind mit der zentralen Rechen- und Regeleinheit 22 über eine erste und eine zweite Signalleitung 24 verbunden. Die Waage 7 liefert entsprechend der jeweiligen Gewichtszu- oder -abnahme Signale über eine weitere Signalleitung 25 an die zentrale Rechen- und Regeleinheit 22.

In die Leitung zwischen dem Ablauf 4 der Zentrifuge 2 und dem Zwischenreservoir 5 ist ein optischer Sensor 26, der über eine dritte Steuerleitung 27, die mit der zentralen Rechen- und Regeleinheit 22 verbunden ist, angeordnet. Mit diesem Sensor 26 wird die Feststoffzunahme der aus dem Durchlaufrotor in das Zwischenreservoir 5 überführten Flüssigkeit beobachtet und bei Überschreiten eines vorgegebenen Wertes der Fluß unterbrochen, da dann davon auszugehen ist, daß entweder die Zentrifuge 2 nicht ordnungsgemäß arbeitet, also der Durchlaufrotor mit Feststoffen überfüllt ist, so daß der Rotor entleert werden muß.

Auch die Ventile 15, 16, 17, 20 und 21 werden von der Rechen- und Regeleinheit 22 angesteuert, ebenso die Drossel 14 über die weitere Steuerleitung 28.

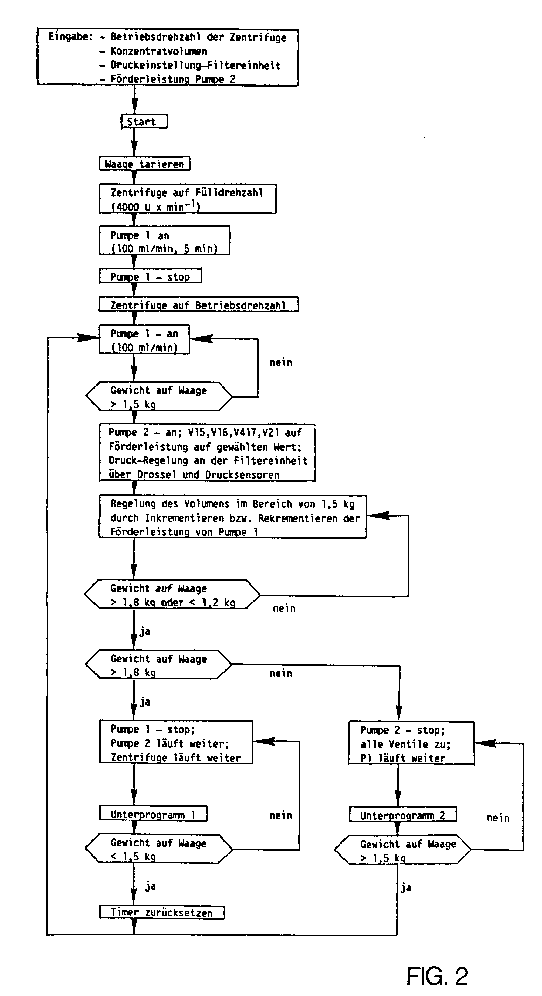

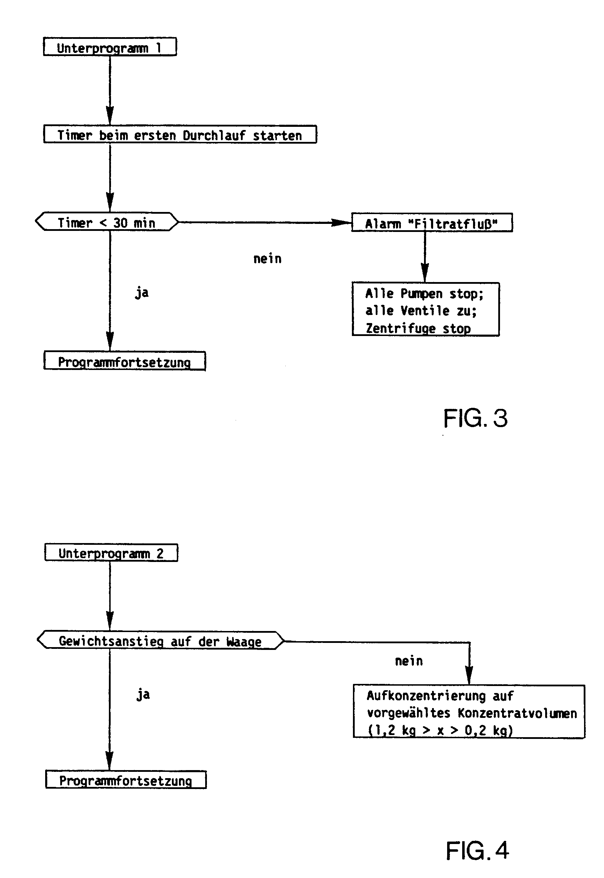

Zu Beginn eines jeden Verfahrenszykluses werden, wie das Ablaufdiagramm nach Figur 2 zeigt, in der zentralen Rechen- und Regeleinheit 22 Eingangsdaten, wie die Betriebsdrehzahl der Zentrifuge 2, das geforderte Konzentratvolumen im Zwischenreservoir 5, die Druckverteilung eingangsseitig bzw. ausgangsseitig der Filtereinheit 9 sowie die Förderleistung der zweiten Pumpe 10, gespeichert. Nach dem anschließenden Start wird automatisch die Waage 7 tariert, oder von der Rechen- und Regeleinheit 22 über die weitere Signalleitung 25 die momentane Anzeige der Waage 7 mit dem darauf stehenden, als Zwischenreservoir 5 dienenden Behälter 6 abgefragt, von dem dann ausgehend die Gewichtszunahme überwacht wird. Nachdem dann die Zentrifuge 2 auf eine Fülldrehzahl, die beispielsweise bei 4000 U/min liegt, hochgefahren ist, mit einer entsprechenden Rückmeldung über die Steuerleitung 23, wird die erste Pumpe 3 über deren Steuerleitung 23 gestartet, die dann beispielsweise mit einer Förderleistung von 100 ml/min für eine Zeitdauer von 5 Minuten ihre Pumptätigkeit aufnimmt, bis der Rotor der Zentrifuge 2 mit aus dem Fermenter 1 entnommener Flüssigkeit gefüllt ist. Anschließend wird die erste Pumpe 3 gestoppt und erneut gestartet, nachdem die Zentrifuge 2 auf ihre Betriebsdrehzahl, die in der Rechen- und Regeleinheit 22 als Eingangsdaten abgespeichert sind, beschleunigt wurde. Die erste Pumpe 3 wird solange betrieben bis die Waage 7 in dem Zwischenreservoir 5 eine Gewichtszunahme der aus der Zentrifuge 2 abgetrennten flüssigen Bestandteile von beispielsweise 1,5 kg ermittelt. Mit Erreichen einer diesem Gewicht entsprechenden Flüssigkeitsmenge in dem Zwischenresevoir 5 bzw. Behälters 6wird über die Steuerleitung 23 sowie über weitere nicht näher gezeigte Signalleitungen, die die Rechen- und Regeleinheit 22 mit den Ventilen 15, 16, 17, 21 verbinden, die zweite Pumpe 10 gestartet und auf eine vorgegebene Förderleistung gebracht sowie die Ventile 15, 16, 17 und 21 geöffnet. Gleichzeitig wird die Druckregelung mit Hilfe der Drossel 14, die über die weitere Steuerleitung 28 mit der zentralen Rechen-und Regeleinheit 22 verbunden ist und der beiden Drucksensoren 13 geregelt. Unter Vorgabe eines oberen und eines unteren Wertes wird das Füllvolumen in dem Zwischenreservoir 5 auf einem annähernd gleichbleibenden Füllstand, beispielsweise 1,5 kg, gehalten, wozu die Förderleistung der ersten Pumpe 3 verändert wird. Falls ein maximaler Füllstand in dem Zwischenreservoir 5, der beispielsweise einem Gewicht von 1,8 kg entspricht, überschritten wird, wird die erste Pumpe 3 gestoppt, während die zweite Pumpe 10 sowie die Zentrifuge 2 weiterläuft. Anschließend wird das Unterprogramm 1, wie es die Figur 3 zeigt durchlaufen. Solange der maximale Füllstand, beispielsweise äquivalent zu einem Gewicht von 1,8 kg, nicht erreicht wird, wird die Förderleistung der zweiten Pumpe 2 gedrosselt oder gestoppt und alle Ventile geschlossen und anschließend das Unterpogramm 2 durchlaufen, wie es die Figur 4 zeigt.

Nach dem Unterprogramm 1 (siehe Figur 3) wird zunächst ein Timer gestartet, der beispielsweise auf einen Wert von 30 Minuten eingestellt wird. Solange diese Zeit noch nicht abgelaufen ist, setzt sich der Prorammablauf entsprechend der Figur 2 fort, indem überprüft wird, ob das Gewicht der Waage 7 unterhalb 1,5 kg liegt. Falls ja, wird der Timer wieder zurückgesetzt und ein Sprung in den vorderen Bereich des Ablaufdiagramme vorgenommen, wobei die erste Pumpe 3 erneut eingeschaltet wird. Falls im Unterprogramm 1 die Zeit des Timers 30 Minuten überschreitet und das Gewicht auf der Waage 7 nicht weiter abnimmt, läßt dies auf eine verstopfte Filtereinheit 9 schließen, so daß Alarm für "Filtratfluß" gegeben und alle Pumpen 3, 10, die Zentrifuge 2 gestoppt und alle Ventile verschlossen werden. Nach einem solchen Alarm muß die Filtereinheit 9 gereinigt werden, beispielsweise durch eine Spülung im Gegenstrom über die Bypass-Leitung 18. Falls in dem Verfahrensablauf nach Figur 2 in der Entscheidungsrombe, in der nachgefragt wird, ob das Gewicht auf der Waage 1,8 kg über- oder unterschreitet, wird, falls das Gewicht unterschritten wird, die zweite Pumpe 10 gestoppt, alle Ventile geschlossen, die erste Pumpe 3 weiterbetrieben und in das Unterprogramm 2 nach Figur 4 übergegangen. Führen diese Maßnahmen zu einem Gewichtsanstieg auf der Waage 7, so wird der Programmablauf nach Figur 2 fortgeführt. Falls kein Gewichtsanstieg auf der Waage 7 zu verzeichnen ist, erfolgt nur noch eine Aufkonzentrierung, wie dies das Unterprogramm 2 in Figur 4 zeigt, auf ein vorgewähltes Konzentratvolumen, wozu ein gesondertes Unterprogramm ablaufen kann. Ist nach dem Unterprogramm 2 das Gewicht auf der Waage 7 größer 1,5 kg ist, wird wiederum in den oberen Bereich des Programmablaufes zurückgesprungen und unmittelbar die zweite Pumpe 10 eingeschaltet und die Ventile geöffnet.

## Patentansprüche

1. Verfahren zum Abtrennen von Feststoffen, z.B. Bakterien, Hefen, tierischen und/oder menschlichen Zellen, aus flüssigen Nährmedien und Anreicherung von in der verbleibenden Flüssigkeit enthaltenen hochmolekularen Substanzen, wobei das flüssige Nährmedium aus einem Fermenter oder Vorratsgefäß entnommen und durch Zentrifugation in seine Feststoffe und flüssigen Bestandteile getrennt wird und in einem weiteren Verfahrensschritt durch Filtrierung in einer Filtereinheit die hochmolekularen Substanzen in den flüssigen Bestandteilen angereichert werden, dadurch gekennzeichnet, daß zentral geregelt der Durchlaufrotor einer Zentrifuge kontinuierlich aus dem Fermenter oder Vorratsgefäß mit flüssigem Nährmedium beschickt wird, bis eine vorgegebene als Sollwert zentral gespeicherte Menge der flüssigen Bestandteile von der Zentrifuge in ein Zwischenreservoir abgegeben ist, und daß diese flüssigen Bestandteile kontinuierlich direkt der Filtereinheit zugeführt werden mit einer anschließenden Rückführung der hochmolekularen Bestandteile in das Zwischenreservoir, wobei der Filtrationsdruck zentral überwacht und in Abhängigkeit der Pumpleistung einer der Filtereinheit zugeordneten Pumpe eingestellt wird und wobei die Beschickung der Zentrifuge innerhalb vorgegebener Werte des momentanen Füllstandes des Zwischenreservoirs geregelt wird.

2. Verfahren nach Anspruch 1, dadurch gekennzeichnet, daß mit steigendem Füllstand in dem Zwischenreservoir die Zuflußrate an flüssigem Nährmedium in die Zentrifuge herabgesetzt wird.

3. Verfahren nach Anspruch 1, dadurch gekennzeichnet, daß mit sinkendem Füllstand in dem Zwischenreservoir die Zuflußrate an flüssigem Nährmedium in die Zentrifuge heraufgesetzt wird.

4. Verfahren nach einem der Ansprüche 1 bis 3, dadurch gekennzeichnet, daß der momentane Füllstand in dem Zwischenreservoir ständig zentral als Istgröße überwacht wird.

5. Verfahren nach Anspruch 4, dadurch gekennzeichnet, daß die Füllstandsüberwachung durch die Bestimmung der Gewichtszu- oder- abnahme des Zwischenreservoirs erfolgt.

6. Verfahren nach Anspruch 5, dadurch gekennzeichnet, daß die Gewichtszu- oder -abnahme des Zwischenreservoirs über eine Waage ermittelt wird, wobei zu Beginn eines Trennvorganges die Waage tariert und/oder der ermittelte Wert zentral gespeichert wird.

7. Verfahren nach einem der Ansprüche 1 bis 6, dadurch gekennzeichnet, daß ausgangsseitig des Durchlaufrotors der Feststoffgehalt der in das Zwischenreservoir überführten flüssigen Bestandteile ermittelt und bei Überschreiten eines vorgegebenen Grenzwertes der Zufluß an flüssigem Nährmedium zu dem Durchlaufrotor unterbrochen wird.

8. Verfahren nach einem der Ansprüche 1 bis 7, dadurch gekennzeichnet, daß die Pumpleistung der der Filtereinheit zugeordneten Pumpe derart geregelt wird, daß ein zentral als Sollwert gespeicherter Filtrationsdruck, verglichen mit dem Druck gebildet aus dem arithmetischen Mittel zwischen dem Druck eingangsseitig der Filtereinheit und dem Druck an dem die hochmolekularen Substanzen abführenden Ausgang der Filtereinheit, nicht überschritten wird.

9. Verfahren nach einem der Ansprüche 1 bis 8, dadurch gekennzeichnet, daß beim Überschreiten einer vorgegebenen, zentralgespeicherten, maximalen Menge an flüssigen Bestandteilen in dem Zwischenreservoir der Fluß eingangsseitig und ausgangsseitig der Filtereinheit unterbrochen wird.

10. Verfahren nach Anspruch 8, dadurch gekennzeichnet, daß der Filtrationsdruck durch die Regelung einer zusätzlichen Drossel an dem die hochmolekularen Substanzen enthaltenden Ausgang der Filtereinheit erfolgt.

11. Verfahren nach einem der Ansprüche 1 bis 10, dadurch gekennzeichnet, daß die Filtereinheit zur Reinigung im Gegenstrom von aus dem Zwischenreservoir entnommener Flüssigkeit durchspült wird.

12. Vorrichtung zur Durchführung des Verfahrens nach einem der Ansprüche 1 bis 11 mit einem das flüssige Nährmedium enthaltenden Fermenter oder Vorratsgefäß, der über eine erste Pumpe strömungsmäßig mit dem Durchlaufrotor einer Zentrifuge verbunden ist, die einen Ablauf aufweist, der in einen Behälter führt, dadurch gekennzeichnet, daß der Behälter als Zwischenreservoir (5) dient und über eine Zuleitung (8) und eine Rückführungsleitung (11) mit einer Ultra-Filtereinheit (9) verbunden ist, wobei in der Zuleitung (8) zu der Filtereinheit (9) eine zweite Pumpe (10) angeordnet ist, und daß die erste Pumpe (3), die zweite Pumpe (10) sowie der Antrieb der Zentrifuge (2) über Steuerleitungen (23) mit einer zentralen Rechen- und Regeleinheit (22) verbunden sind.

13. Vorrichtung nach Anspruch 12, dadurch gekennzeichnet, daß in der Zuleitung (8) zu der Filtereinheit (9) sowie in der Rückführungsleitung (11) zwischen der Filtereinheit (9) und dem Zwischenreservoir (5) jeweils ein Drucksensor (13) angeordnet ist, die über eine erste und eine zweite Signalleitung (24) mit der Rechen- und Regeleinheit (22)verbunden sind.

14. Vorrichtung nach Anspruch 12 oder 13, dadurch gekennzeichnet, daß das Zwischenreservoir (5) auf einer elektronischen Waage (7) steht, die über eine weitere Signalleitung (25) mit der Rechen- und Regeleinheit (22) verbunden ist.

15. Vorrichtung nach einem der Ansprüche 12 bis 14, dadurch gekennzeichnet, daß ausgangsseitig des Durchlaufrotors in den Ablauf (4) zu dem Zwischenreservoir (5) ein optischer Sensor (26) angeordnet ist, der über eine dritte Steuerleitung (26) mit der Rechen- und Regeleinheit (22) verbunden ist.

16. Vorrichtung nach einem der Ansprüche 12 bis 15, dadurch gekennzeichnet, daß in der Rückführungsleitung (11) zwischen der Filtereinheit (9) und dem Zwischenreservoir (5) eine Drossel (14) angeordnet ist, die über eine weitere Steuerleitung (28) mit der Rechen- und Regeleinheit (22) verbunden ist.

17. Vorrichtung nach einem der Ansprüche 12 bis 14, dadurch gekennzeichnet, daß in der Zuleitung (8) zu der Filtereinheit (9) sowie in der Rückführungsleitung (11) zwischen der Filtereinheit (9) und dem Zwischenreservoir (5) jeweils ein Absperrventil (15, 16) angeordnet ist, die über die Rechen- und Regeleinheit (22) ansteuerbar sind.

18. Vorrichtung nach einem der Ansprüche 12 bis 17, dadurch gekennzeichnet, daß die Filtereinheit (9) einen weiteren Ausgang für von hochmolekularen Substanzen gereinigte Flüssigkeit aufweist, der über ein weiteres, über die Rechen- und Regeleinheit (22) ansteuerbares Absperrventil (17) verschließbar ist.

19. Vorrichtung nach einem der Ansprüche 12 bis 18, dadurch gekennzeichnet, daß ausgangsseitig der zweiten Pumpe (10) eine Bypass-Leitung (18) abzweigt, die über ein darin eingesetztes Bypass-Ventil (20) absperrbar ist und der Filtereinheit (9) im Gegenstrom flüssige Bestandteile zuführt.

20. Vorrichtung nach Anspruch 19, dadurch gekennzeichnet, daß ausgangsseitig der zweiten Pumpe (10) in der Zuleitung (8) zu der Filtereinheit (9) nach der Abzweigung der Bypass-Leitung (18) ein Rückstau-Ventil (20) angeordnet ist, das entgegen der Pumprichtung der zweiten Pumpe (10) wirkt.

## Claims

1. A method for the separation of solids, e.g. bacteria, yeasts, animal and/or human cells, from liquid nutrient media and the enrichment of high-molecular substances contained in the remaining liquid, in which the liquid nutrient medium is taken from a fermenter or storage vessel and is separated by centrifuging into its solids and liquid components, and in a further process step through filtering in a filter unit, the high-molecular substances in the liquid components are enriched, characterised in that the continuous rotor of a centrifuge, in a centrally regulated manner, is charged continuously with liquid nutrient medium from the fermenter or storage vessel, until a given, centrally stored amount of the liquid components, as nominal value, is emitted from the centrifuge into an intermediate reservoir, and that these liquid components are supplied continuously directly to the filter unit with a subsequent return of the high-molecular components into the intermediate reservoir, in which the filtration pressure is monitored centrally and is adjusted as a function of the pumping capacity of a pump associated with the filter unit and in which the charging of the centrifuge is regulated within given values of the instantaneous filling level of the intermediate reservoir.

2. A method according to Claim 1, characterised in that with a rising filling level in the intermediate reservoir, the inflow rate of liquid nutrient medium into the centrifuge is reduced.

3. A method according to Claim 1, characterised in that with a falling filling level in the intermediate reservoir, the inflow rate of liquid nutrient medium into the centrifuge is increased.

4. A method according to one of Claims 1 to 3, characterised in that the instantaneous filling level in the intermediate reservoir is continuously monitored centrally as actual value.

5. A method according to Claim 4, characterised in that the monitoring of the filling level takes place by determining the increase or decrease in weight of the intermediate reservoir.

6. A method according to Claim 5, characterised in that the increase or decrease in weight of the intermediate reservoir is determined by means of a balance, in which at the beginning of a separation process the balance is tared and/or the determined value is stored centrally.

7. A method according to one of Claims 1 to 6, characterised in that at the output side of the continuous rotor the solids content of the liquid components, which are transferred into the intermediate reservoir, is determined and on exceeding a given threshold value, the inflow of liquid nutrient medium to the continuous motor is interrupted.

8. A method according to one of Claims 1 to 7, characterised in that the pumping capacity of the pump, which is associated with the filter unit, is regulated such that a filtration pressure, centrally stored as nominal value, compared with the pressure formed from the arithmetic mean between the pressure at the input side of the filter unit and the pressure at the output of the filter unit, carrying away the high-molecular substances, is not exceeded.

9. A method according to one of Claims 1 to 8, characterised in that on exceeding a given, centrally stored, maximum amount of liquid components in the intermediate reservoir, the flow on the input side and on the output side of the filter unit is interrupted.

10. A method according to Claim 8, characterised in that the filtration pressure takes place through the regulating of an additional throttle at the outlet of the filter unit containing the high-molecular substances.

11. A method according to one of Claims 1 to 10, characterised in that the filter unit is flushed for cleaning in counterflow by liquid taken from the intermediate reservoir.

12. A device to carry out the method according to one of Claims 1 to 11, with a fermenter or storage vessel containing the liquid nutrient medium, which is connected via a first pump, in terms of flow, with the continuous rotor of a centrifuge, which has an outlet which leads into a container, characterised in that the container serves as intermediate reservoir (5) and is connected via a feed line (8) and a return line (11) with an ultra filter unit (9), in which in the feed line (8) to the filter unit (9) a second pump (10) is arranged, and that the first pump (3), the second pump (10) and the drive of the centrifuge (2) are connected via control lines (23) with a central digital- and regulating unit (22).

13. A device according to Claim 12, characterised in that in the feed line (8) to the filter unit (9) and also in the return line (11) between the filter unit (9) and the intermediate reservoir (5) in each case a pressure sensor (13) is arranged, which are connected via a first and a second signal line (24) with the digital- and regulating unit (22).

14. A device according to Claim 12 or 13, characterised in that the intermediate reservoir (5) stands on an electronic balance (7), which is connected via a further signal line (25) with the digital- and regulating unit (22).

15. A device acccording to one of Claims 12 to 14, characterised in that at the outlet side of the continuous rotor into the outlet (4) to the intermediate reservoir (5) an optical sensor (26) is arranged, which is connected via a third control line(26) with the digital- and regulating unit (22).

16. A device according to one of Claims 12 to 15, characterised in that in the return line (11) between the filter unit (9) and the intermediate reservoir (5) a throttle (14) is arranged, which is connected via a further control line (28) with the digital- and regulating unit (22).

17. A device according to one of Claims 12 to 14, characterised in that in the feed line (8) to the filter unit (9) and also in the return line (11) between the filter unit (9) and the intermediate reservoir (5) in each case a shut-off valve (15,16) is arranged, which are controllable via the digital- and regulating unit (22).

18. A device according to one of Claims 12 to 17, characterised in that the filter unit (9) has a further outlet for liquid cleaned of high-molecular substances, which is able to be closed by means of a further shut-off valve (17) which is controllable via the digital- and regulating unit (22).

19. A device according to one of Claims 12 to 18, characterised in that at the outlet side of the second pump (10) a by-pass line (18) branches off, which is able to be shut off by a by-pass valve (20) inserted therein, and feeds liquid components to the filter unit (9) in counter current.

20. A device according to Claim 19, characterised in that at the outlet side of the second pump (10) in the feed line (8) to the filter unit (9) after the branch of the by-pass line (18) a backwash valve (20) is arranged, which acts against the pumping direction of the second pump (10).

## Revendications

1. Procédé pour séparer des matières solides, par exemple des bactéries, des levures, des cellules animales et/ou humaines, à partir de milieux nutritifs liquides et pour enrichir les substances de haut poids moléculaire contenues dans le liquide restant, le milieu nutritif liquide étant prélevé dans un fermenteur ou un récipient de stockage et étant scindé par centrifugation en ses matières solides et en ses constituants liquides, et les substances de haut poids moléculaire présentes dans les constituants liquides étant enrichies dans une étape de procédé ultérieur par filtration dans une unité de filtration, caractérisé en ce que le rotor continu d'une centrifugeuse est alimenté en continu en milieu nutritif liquide à partir du fermenteur ou du récipient de stockage, grâce à une régulation centrale, jusqu'à ce qu'une quantité prédéterminée de constituants liquides, stockée dans une mémoire centrale sous forme de valeur de consigne, soit délivrée par la centrifugeuse dans un réservoir intermédiaire, et en ce que ces constituants liquides sont envoyés directement et en continu à l'unité de filtration, avec recyclage subséquent des constituants de haut poids moléculaire dans le réservoir intermédiaire, la pression de filtration étant surveillée par une unité centrale et étant réglée en fonction du débit d'une pompe associée à l'unité de filtration, et l'alimentation de la centrifugeuse étant régulée en fonction d'une plage prédéterminée de valeurs du niveau de remplissage momentané du réservoir intermédiaire.

2. Procédé selon la revendication 1, caractérisé en ce que le débit d'alimentation en milieu nutritif liquide de la centrifugeuse diminue lorsque le niveau de remplissage du réservoir intermédiaire augmente.

3. Procédé selon la revendication 1, caractérisé en ce que, lorsque le niveau de remplissage du réservoir intermédiaire baisse, le débit d'alimentation en milieu nutritif liquide de la centrifugeuse augmente.

4. Procédé selon l'une des revendications 1 à 3, caractérisé en ce que le niveau de remplissage momentané du réservoir intermédiaire est surveillé en continu sous forme d'une grandeur réelle par une unité centrale.

5. Procédé selon la revendication 4, caractérisé en ce que la surveillance du niveu de remplissage est réalisée par la détermination de l'augmentation ou de la diminution du poids du réservoir intermédiaire.

6. Procédé selon la revendication 5, caractérisé en ce que l'augmentation ou la diminution du poids du réservoir intermédiaire est déterminée par une balance, la balance étant tarée et/ou la valeur déterminée étant stockée dans une unité centrale au début d'un processus de séparation.

7. Procédé selon l'une des revendications 1 à 6, caractérisé en ce que du côté de la sortie du rotor continu la teneur en matières solides des constituants liquides transmis dans le réservoir intermédiaire est déterminée et lorsqu'une valeur limite prédéterminée est dépassée, l'alimentation en milieu nutritif liquide du rotor continu est interrompue.

8. Procédé selon l'une des revendications 1 à 7, caractérisé en ce que le débit de la pompe associée à l'unité de filtration est régulé de telle manière qu'une pression de filtration stockée sous forme de valeur de consigne dans une unité centrale, comparée à la pression obtenue à partir de la moyenne arithmétique entre la pression du côté de l'entrée de l'unité de filtration et la pression à la sortie de l'unité de filtration qui évacue les substances de haut moléculaire, ne soit pas dépassée.

9. Procédé selon l'une des revendications 1 à 8, caractérisé en ce que, en cas de dépassement d'une quantité maximale prédéterminée, stockée dans une unité centrale, de constituants liquides dans le réservoir intermédiaire, le débit du côté de l'entrée et du côté de la sortie de l'unité de filtration est interrompu.

10. Procédé selon la revendication 8, caractérisé en ce que la pression de filtration résulte de la régulation d'un étranglement supplémentaire au niveau de la sortie de l'unité de filtration qui contient les substances de haut poids moléculaire.

11. Procédé selon l'une des revendications 1 à 10, caractérisé en ce que l'unité de filtration est lavée à contre-courant par du liquide prélevé dans le réservoir intermédiaire en vue de son nettoyage.

12. Dispositif pour la mise en oeuvre du procédé selon l'une des revendications 1 à 11, comportant un fermenteur ou un récipient de stockage contenant le milieu nutritif liquide, qui est relié par écoulement, par l'intermédiaire d'une première pompe, au rotor continu d'une centrifugeuse qui présente une sortie qui conduit à un récipient, caractérisé en ce que le récipient sert de réservoir intermédiaire (5) et est relié par l'intermédiaire d'une conduite d'alimentation (8) et d'une conduite de recyclage (11) à une unité d'ultrafiltration (9), une seconde pompe (10) étant prévue dans la conduite d'alimentation (8) qui aboutit à l'unité de filtration (9), et en ce que la première pompe (3), la seconde pompe (10) et le dispositif d'entraînement de la centrifugeuse (2) sont reliés par des conduites de commande (23) à une unité centrale de calcul et de régulation (22).

13. Dispositif selon la revendication 12, caractérisé en ce qu'il est prévu dans la conduite d'alimentation (8) de l'unité de filtration (9) et dans la conduite de recyclage (11) entre l'unité de filtration (9) et le réservoir intermédiaire (5) des capteurs de pression (13) qui sont reliés à l'unité de calcul et de régulation (22) par une première et une seconde conduites de signaux (24).

14. Dispositif selon la revendication 12 ou 13, caractérisé en ce que le réservoir intermédiaire (5) est disposé sur une balance électronique (7) qui est reliée à l'unité de calcul et de régulation (22) par l'intermédiaire d'une autre conduite de signaux (25).

15. Dispositif selon l'une des revendications 12 à 14, caractérisé en ce qu'il est prévu du côté de la sortie du rotor continu, dans la sortie (4) conduisant au réservoir intermédiaire (5), un capteur optique (26) qui est relié à l'unité de calcul et de régulation (22) par l'intermédiaire d'une troisième conduite de commande (26).

16. Dispositif selon l'une des revendications 12 à 15, caractérisé en ce qu'il est prévu dans la conduite de recyclage (11) entre l'unité de filtration (9) et le réservoir intermédiaire (S) un étranglement (14) qui est relié à l'unité de calcul et de régulation (22) par l'intermédiaire d'une autre conduite de commande (28).

17. Dispositif selon l'une des revendications 12 à 14, caractérisé en ce qu'il est prévu dans la conduite d'alimentation (8) de l'unité de filtration (9) et dans la conduite de recyclage (11) entre l'unité de filtration (9) et le réservoir intermédiaire (5) des vannes d'arrêt (15, 16) qui peuvent être commandées par l'intermédiaire de l'unité de calcul et de régulation (22).

18. Dispositif selon l'une des revendications 12 à 17, caractérisé en ce que l'unité de filtration (9) comporte une autre sortie pour le liquide débarrassé des substances de haut poids moléculaire, qui peut être fermée par l'intermédiaire d'une autre vanne d'arrêt (17) qui peut être commandée par l'unité de calcul et de régulation (22).

19. Dispositif selon l'une des revendications 12 à 18, caractérisé en ce que, du côté refoulement de la seconde pompe (10), prend naissance une conduite de dérivation (18) qui peut être fermée par l'intermédiaire d'une vanne de dérivation (20) prévue dans celle-ci et qui fournit des constituants liquides à l'unité de filtration (9) à contre-courant.

20. Dispositif selon la revendication 19, caractérisé en ce qu il est prévu du côté refoulement de la deuxième pompe (10) dans la conduite d'alimentation (8) de l'unité de filtration (9) en aval de la naissance de la conduite de dérivation (18) une vanne antiretour (20) qui agit en sens inverse du sens de pompage de la deuxième pompe (10).
